# EUROPEAN PATENT APPLICATION

(11) **EP 1 557 158 A1**
(43) Date of publication of application: **27.07.2005**
(21) Application number: 03769514.5
(22) Date of filing: 31.10.2003
(51) Int. Cl.: A61K 7/16

(54) **ORAL ANTIMICROBIAL COMPOSITION AND USE THEREOF IN THE TREATMENT OF DENTAL PLAQUE AND GINGIVITIS**

(30) Priority: 31.10.2002 ES 200202506
(71) Applicant: Segura Ludena, Miguel Angel, 08860 Castelldefels (Barcelona) (ES)
(72) Inventor: Segura Ludena, Miguel Angel, 08860 Castelldefels (Barcelona) (ES)
(74) Representative: Carvajal y Urquijo, Isabel
(86) International application number: PCT/ES2003/000562
(87) International publication number: WO 2004/039342

(57) **Abstract**

An antimicrobial oral composition and use thereof in treating dental plaque and gingivitis, **characterized in that** said composition comprises:
- Active carbon as a surfactant: from 0.5% to 2% by weight.
- Carbopol 934 as a binding agent: from 2% to 3% by weight.
- Flavouring agents: from 0.05% to 6% by weight.
- Sweeteners: 0.01% to 40% by weight.

## Description

### Field of the Invention

The present invention refers to an antimicrobial oral composition also incorporating activated carbon and the use thereof in treating dental plaque, gingivitis, periodontitis and other dental diseases.

### State of the Art

As is well known, dental plaque is a dense, heterogeneous and non-calcified bacterial mass which adheres firmly to the tooth surface. The bacteria contained in plaque have pathogenic activity of various degrees and are partly responsible for dental caries, gingivitis, halitosis and periodontal disease.

*Streptococcus mutans* is one of the bacteria found in dental plaque and has a high cariogenic potential in different laboratory animals.

*Actinomyces viscosus,* other dental plaque bacteria, has been associated with gingivitis and root surface caries. Obviously, the elimination or inhibition of plaque formation will significantly reduce the occurrence of these diseases.

Plaque is generally eliminated by the use of mechanical cleaning using an abrasive dentifrice, by means of dental floss or using an antibacterial (anti-plaque) mouth rinse. However, plaque deposited between the teeth is difficult to eliminate by means of mechanical cleaning and dental floss does not eliminate the plaque which is located on the gingival edge. Anti-plaque rinses serve as an accessory to the mechanical elimination of plaque. Until now, an anti-plaque rinse that may substitute mechanical elimination thereof has not been discovered. Rinses are often ineffective in the last development phases of plaque, and they are useless if the plaque has calcified forming calculi or tartar.

Plaque formation on a clean tooth generally starts with the formation of a film or cuticle made up of saliva constituents. The film is an amorphous and membranous layer covering the enamel surface and is made up of saliva glycoproteins, polypeptides and other saliva constituents that have been selectively absorbed on the tooth surface. The film is normally free of bacteria. The film is formed in the minutes following tooth cleaning and the absorbed materials are finally converted into a highly insoluble layer. Subsequently, an initial adherence of bacteria on the acquired film takes place. These bacteria then produce extracellular polysaccharides, called glucanes, from sucrose catalysed by the glucosyltransferase enzyme which aids in the trapping and adherence of other bacterias.

The cariogenic potential of *S*. *mutans,* for example, is associated with its dental plaque forming ability and this ability depends on extracellular polysaccharide synthesis from sucrose.

As well as the initial adherence and glucosyltransferase catalysis aiding in adherence, the coaggregation of several bacteria species takes place in which specific bacteria linking with one another, synthesising polymers linking similar and different cells, although there are some species which do not coaggregate.

On the other hand, activated carbon is characterised by its thousands of small pores which consequently confer it a great surface area.

The total inner surface of said pores in the more common types of carbon ranges from 500 to 1,400 m²/g. Given that the efficacy of an adsorbent is directly related to its total surface, activated carbons are very effective adsorbents precisely because of the great total surface they have.

In the adsorption of bigger molecules in liquids, pores of 2 to 10 nanometres wide are normally used.

Adsorption is a natural phenomenon by means of which the molecules of a liquid or a gas are fixed onto the inner or outer surface of a solid.

On the other hand, on the surface layer these molecules are subject to unstable forces, due to their not being completely surrounded by other molecules. As these molecules aim to reach a state of equilibrium, a surface tension or energy is created. This surface energy is an attraction force and if it is strong enough, it may exceed the energy of the other molecules that might adhere or be adsorbed on the surface. These forces are known as 'London dispersion forces'.

In an effort to eliminate dental plaque, agents incorporated into oral preparations inhibiting the formation of such plaque more than inducing the elimination thereof as previously set forth have been disclosed in the prior state of the art.

US patent 4,117,107 discloses a method for delaying film and plaque formation that includes putting the plaque formation and growth sites in contact with a dental preparation containing certain fatty acid amido betaines.

Likewise, US patent 4,130,637 provides betaine compounds derived from 50 higher alkyl methyl carboxylic acid quaternary ammonium compounds, which are effective in controlling dental plaque without producing an aesthetically unacceptable discolouration of the teeth.

US patent 4,360,515 provides compounds for preventing dental plaque from fixing upon the teeth comprising certain sulphonated alkoxy naphthalenes and pharmaceutically acceptable salts thereof.

US patent 3,981,989 deals with an oral preparation for preventing dental caries containing dextranase stabilised with a special protein such as gelatine or peptone. Non-ionic surfactants with an anionic surfactant may also be added to stabilise dextranase. Sodium acyltaurate is a suitable anionic surfactant and is preferably present at a ratio of 0.5% to 5% by weight of the oral preparation.

British patent 990,957 discloses a toothpaste containing strontium ions for treating hypersensitive dentins containing, amongst others, compatible surfactant foaming agents including fatty acid condensates and N-methyltaurine.

With this background, it is obvious that it would be desirable to be able to have a new agent solving the drawbacks of the aforementioned state of the art.

### Detailed Description of the Invention

The present invention provides an effective plaque-inhibiting composition containing a surfactant, the activated carbon, which acts to prevent the initial adherence of bacteria and extracellular polysaccharide formation from bacteria catalysed by glucosyltransferase in plaque formation and, therefore, substantially inhibit formation thereof. Surfactants are effective at very low concentrations and are suitable for their use in oral compositions.

In short, the invention provides an oral preparation or composition, such as an oral wash or rinse solution, a toothpaste and the like, containing a surfactant which acts by inhibiting plaque formation by means of inhibiting the initial adherence of bacteria to dentine surfaces and further inhibiting glucosyltransferase catalysis to prevent extracellular polysaccharide synthesis from sucrose, also acting in the prevention of caries or regression thereof during their formation.

Due to the activated carbon adsorption capacity it is very useful in capturing the acids attacking the tooth enamel due to the bacterial decomposition produced therein, likewise and due to its ductile and slightly abrasive capacity, it is very useful in eliminating dental plaque and it also counteracts the interaction/attack produced by the caries virus on tooth enamel. In short, this is a simple but at the same time peculiar finding in the oral cleaning perspective which leads its users to clean the tooth surface in a novel and complete manner; it is also useful and effective as a bacterial and/or viral decomposition acid inhibitor, clearly opposed to the advance of periodontal processes known as gingivitis or advanced periodontitis (pyorrhea), caries and tartar (dental plaque).
Qualitative formulation: Excipient: deionised water, Carbopol 934, Active Ingredient: activated carbon of mineral or plant origin.

It has been demonstrated in the clinical trial phase that the application of the gel with a non-moistened toothbrush is more effective regarding the short period in which visible results in treating dental plaque and cariogenic processes are achieved, also and as a result of said empirical trials in the action groups based on the preparation, a greater action of the product has been observed using electromechanical toothbrushes, when the toothbrushes are previously moistened, however, a reduction in the effectiveness of the compound is observed that may be assessed in an approximately 20% prolongation in the treatment time with the surfactant.

The mentioned surfactant in the performed field studies is activated carbon in fine powder form, the usual mean thickness of which is 98-100% < 150 microns 60-65% < 45 microns.

According to the present invention, a gel is provided in an oral medium, the surfactant of which is activated carbon in the aforementioned ratios.

The anti-plaque surfactants of the invention can be easily incorporated into oral compositions containing water or water/alcohol such as solutions for mouth washes, aerosols, rinses, toothpastes, dental creams, gels or dental powders.

The surfactant is preferably present in amounts from about 0.5% to about 2% of the total weight.

In one manner of carrying out the invention, the oral composition may be a liquid such as a mouth wash solution, an aerosol or a rinse. In such compositions the carrier is usually an alcohol/water mixture. The ratio of total water to alcohol in a mouth wash preparation is generally within the range between about 1:1 and about 20:1, preferably between 30 about 3:1 and about 20:1, and more preferably between about 3:1 and about 10:1 by weight. The total amount of water/alcohol mixture in a mouth wash preparation is normally within the range of about 45% to about 82.5% by weight of the composition. The pH value of such mouth wash preparation is generally about 4 to about 9, and preferably about 5. A pH lower than 4 is irritating for the oral cavity and a pH exceeding 9 causes an unpleasant sensation in the mouth.

Carbopol is preferably present in amounts from about 2% up to about 3% of the total weight.

Fluoride compounds may be present in the oral preparations of this invention. These compounds may be slightly water soluble or completely water soluble and are
characterized by their ability to release fluoride ions or ions containing fluoride in water. Usual compounds that provide fluoride are inorganic fluoride salts such as soluble alkaline metal salts, alkaline-earth metal salts, and heavy metal salts, for example, sodium fluoride, potassium fluoride, ammonium fluoride, copper fluoride, zinc fluoride, tin fluoride, barium fluoride, sodium fluorosilicate, ammonium silicofluoride, sodium fluorozirconate, sodium monofluorophosphate, aluminium mono and difluorophosphate and sodium pyrophosphate and fluorinated calcium.

Alkaline metal salts, tin fluorides and monofluorophosphates such as sodium and tin fluorides, sodium monofluorophosphate and mixtures thereof, are preferred.

In an oral liquid composition such as a mouth wash solution, the compound providing fluoride is generally present in a sufficient amount as to release up to about 0.15%, preferably from about 0.001% to about 0.1%, and more preferably from about 0.001% to about 0.05% of fluoride by weight of the preparation.

The oral composition may also contain additional flavouring agents and dyes.

In the case of using auxiliary sweeteners, the present invention contemplates inclusion of those sweeteners that are well known in the art, including natural and artificial sweeteners. Thus, additional sweeteners may be chosen in small amounts from the following non-limiting list:
- Water soluble sweetening agents such as monosaccharides, disaccharides and polysaccharides, such as xylose, ribose, glucose, manose, galactose, fructose, dextrose, sucrose, maltose, starch or partially hydrogenated corn syrup solids and saccharide alcohols such as sorbitol, xylitol, manitol and mixtures thereof.
- Water soluble artificial sweeteners such as soluble cyclamate salts and the like.
- Dipeptide-based sweeteners such as L-phenylalanin methyl ester and materials disclosed in US patent 3,492,131 and the like. The amount of sweetener will vary with the desired amount of the sweeteners chosen for a particular oral preparation. This amount will normally be from a 0.01% up to about 40% by weight. The previously described water soluble natural sweeteners are preferably used in amounts from about 5% up to about 40% by weight, and more preferably from about 10% up to about 20% by weight of the final composition.

In contrast, the artificial sweeteners described are used in amounts from about 0.005% up to about 5.0%, and more preferably from about 0.059 up to about 2.5% by weight of the final composition.

These amounts are usually necessary in order to reach a desired level of sweetness, independently from the level of flavour reached with the flavouring agents.

Suitable flavouring agents include natural and artificial flavours, and mint flavours such as peppermint and spearmint. Citric flavours such as orange or lemon, various fruit flavours, both individually as well as mixed, and the like, are contemplated. The flavouring agents are generally used in amounts that will vary depending on the individual flavour and may, for example, range in amounts from about 0.05% up to about 6% by weight of the final composition.

Due to the absorbing character not only of organic and inorganic materials the surfactant and abrasive agent, known as activated carbon due its intense black colour, makes it unnecessary to add further colourings as the black colour of the activated carbon absorbs all initial colouring.

The oral compositions may also be of a substantially solid or pasty character such as dental creams, toothpastes, dental powders or gels. Solid or pasty oral preparations contain polishing materials, although in this case the surfactant such as the activated carbon performs said function. Usual polishing materials are abrasive materials in particles having particle sizes of up to about 98-100% < 150 microns 60-65% < 45 microns. Water insoluble sodium metaphosphate, potassium metaphosphate, tricalcium phosphate, dihydrated calcium phosphate, calcium pyrophosphate, magnesium orthophosphate, trimagnesium phosphate, calcium carbonate, alumina, aluminium silicate, zirconium silicates, silica, bentonite, and mixtures thereof may be included within these materials in an illustrative and non-limiting manner.

The water content for toothpastes and dental creams is about 25% to 50% by weight.

Furthermore, the orally acceptable carrier may include optional components, such as detergents, flavouring agents, sweetening agents, anticaries agents, as well as the fluoride ion source, agents preventing calculi formation, teeth desensitising agents, dyes, preservatives and pigments. The useful detergents include water soluble alkyl sulphate salts having from 10 to 18 carbon atoms in the alkyl radical, such as sodium laurylsulphate, but other anionic detergents as well as non-anionic, bipolar, cationic and amphoteric detergents may also be used. An agent preventing calculi formation comprises a water soluble alkaline metal polyphosphate, for example sodium tripolyphosphate and the like, a fluoride ion source and, if so desired, a synthetic linear polymeric polycarboxylate with a molecular weight from 2000 to one million.

In clear gels, colloidal silica and aluminium silicate complexes of or alkaline metal polishing agents are preferred as they have refraction indexes close to the refraction indexes of gelling agent liquid systems commonly used in toothpastes.

The anti-plaque oral compositions of the present invention are generally prepared as follows. If a sweetener were added, this is dissolved and mixed until it dissolves, then sufficient water, alcohol or mixtures thereof are added with the mixture until the final volume of the solution is reached. When dyes, artificial sweeteners and similar additives are included in the composition, these are added at the same time as the sweetener. The anti-plaque surfactant may also be added as a final ingredient.

In the formulation of the dentifrice preparation according to the present invention, apart from the cariogenic processes and the dental abrasive, the antimicrobial anti-plaque and anti-gingivitis agent is made up of a single surfactant which is activated carbon of plant and/or mineral origin, which is incorporated in an orally acceptable dental carrier which may be anhydrous, but which is preferably an orally acceptable aqueous dental carrier, in order to form a semi-solid material which can be extruded and is stable in solid storage such as a toothpaste or tooth gel.

As is conventionally the case, the orally acceptable dental carrier will comprise a binding and/or thickening carrier, such as natural and synthetic gums, for example, carbopol, xanthan gum, carrageenans, alginates, cellulose ethers and ethers or silica. A suitable wetting agent such as glycerine, sorbitol, propylene glycol or polyethylene glycol, is used in the formulation of the orally acceptable and preferred aqueous dental carrier. Conventional manufacturing techniques are used to manufacture the tooth gel according to the present invention, which will normally have a pH from 4 to 9.

The tooth gel of the present invention is used by applying the tooth gel to the teeth, preferably using this without residual moisture.

Rubbing the teeth with the tooth gel of the present invention, dental plaque formation and gingivitis is reduced and cariogenic processes are stopped.

### Detailed Description of an Embodiment

The invention will be illustrated below by the following non-limiting example of the scope thereof. All the parts and percentages mentioned in the specification and in the enclosed claims are by weight with respect to the total weight of the oral composition, unless otherwise specified.

A tooth gel was prepared from the following components for a total capacity of 100 ml:

| **INGREDIENTS** | **%W/W** |
|---|---|
| Carbopol 934 | 2.000 |
| Activated carbon | 1.500 |
| Menthol | 0.250 |
| Citrus mint flavour | 1.000 |
| Sodium saccharine | 0.250 |
| Sodium benzoate | 0.200 |
| Deionised water excipient | s.q. |
| | 100.000% |

A random, parallel three-month group study was performed to compare the antimicrobial dentifrice of the invention with a control dentifrice containing sodium fluoride but without the anti-plaque and anti-gingivitis and anti-cariogenic process agent of the tooth gel-(mineral and/or plant activated carbon).

Adult subjects were examined to check for the presence of supragingival plaque and mild gingivitis. Subjects were admitted to the study based on a minimum gingivitis index of 0.5 according to the Lobene non-invasive modification of the Loe-Silness index. Plaque was measured using the Tursky Quigley-Hein modification. A total of 57 subjects were introduced in the study.

Qualified subjects were assigned dentifrice groups by random permutations of two.

Thirty subjects were assigned to the tooth gel of the invention and 27 to the control tooth gel. All subjects received a complete oral prophylaxis in order to eliminate any supragingival plaque, calculi and extrinsic dental stains.

The subjects were then provided with the assigned tooth gel, a new soft bristle toothbrush and instructions to brush twice a day for one minute. The use of other dentifrices, mouthwashes, mouth irrigation equipment and tooth picks was prohibited during the trial. Plaque and gingivitis were evaluated after 12 weeks use.

The results of this trial demonstrated that both plaque and gingivitis were delayed to a greater extent in the subjects using the gel of the invention in comparison to those using the conventional control fluoride tooth gel. The effect on plaque and gingivitis was visible after only six weeks of use, and after 12 weeks, the difference between the active and control groups was 35% with respect to gingivitis and 57% with respect to plaque. The difference in plaque and gingivitis evaluations after a 12 week treatment reached statistical significance (P 0.08, P 0.03, respectively) in a one-tailed t-test.

The subjects that used the tooth gel of the invention found the flavour acceptable.

It is concluded from this trial that regular use of the antimicrobial tooth gel of the invention reduces dental plaque by 57% and gingivitis by 35% in comparison to a conventional fluoride tooth gel.

## Claims

1. An oral antimicrobial composition, **characterized in that** it comprises:
- Activated carbon as a surfactant: from 0.5% to 2% by weight.
- Carbopol 934 as a binding agent: from 2% to 3% by weight.
- Flavouring agents: form 0.05% to 6% by weight.
- Sweeteners: 0.01 to 40% by weight.

2. An oral composition according to claim 1, **characterized in that** it comprises an effective amount of compounds providing fluoride.

3. An oral composition according to claims 1 and 2, **characterized in that** said compounds providing fluoride are found in amounts of up to 0.15% with respect to the total weight, and preferably from 0.001% to 0.05% with respect to the total weight.

4. An oral composition according to claim 1, **characterized in that** the sweeteners are preferably found in amounts from 5% to 40% with respect to the total weight, and more preferably between 10% and 20% with respect to the total weight.

5. An oral composition according to claim 1, **characterized in that** in incorporates dyes.

6. An oral composition according to claim 1, **characterized in that** it incorporates teeth desensitising agents.

7. An oral composition according to claim 1, **characterized in that** it incorporates detergents.

8. An oral composition according to claim 1, **characterized in that** it incorporates an agent preventing calculi formation.

9. Use of the previously claimed antimicrobial composition in the elaboration of toothpastes, tooth gels, mouth rinses and the like, for treating dental plaque and gingivitis.
